Europäisches Patentamt

European Patent Office

Office européen des brevets

(11) Veröffentlichungsnummer: **0 241 809**
**B1**

(12) **EUROPÄISCHE PATENTSCHRIFT**

(45) Veröffentlichungstag der Patentschrift:
08.08.90

(51) Int. Cl.⁵: **A61K 31/13**

(21) Anmeldenummer: **87104818.7**

(22) Anmeldetag: **01.04.87**

(54) Synergistische Kombination von Amantadin und Selegilin.

(30) Priorität: **16.04.86 DE 3612702**

(43) Veröffentlichungstag der Anmeldung:
**21.10.87 Patentblatt 87/43**

(45) Bekanntmachung des Hinweises auf die Patenterteilung:
**08.08.90 Patentblatt 90/32**

(84) Benannte Vertragsstaaten:
**AT BE CH DE ES FR GB GR IT LI LU NL SE**

(56) Entgegenhaltungen:
**DE-A- 2 253 130**

(73) Patentinhaber: **ASTA Pharma Aktiengesellschaft,
Weismüllerstrasse 45, D-6000 Frankfurt am Main 1(DE)**

(72) Erfinder: **Reischig, Dirk, Dr., Mittlerer Reisberg 15,
D-6380 Bad Homburg(DE)**
Erfinder: **Hettche, Helmut, Dr., Buchrainweg 65,
D-6050 Offenbach(DE)**
Erfinder: **Brade, Wolfgang, Dr., Im Baumgarten 2,
D-6393 Wehrheim 4(DE)**

## Beschreibung

Amantadin ist ein Virusstatikum sowie ein Anti-Parkinsonmittel.

Die chemische Bezeichnung ist 1-Amino-adamantan.

Selegilin ist ein Antidepressivum sowie ein Appetizügler. Die chemische Bezeichnung ist N-(2-Phenylisopropyl)-N-methyl-N-propin-(2)-yl-amin.

Es wurde nun gefunden, daß die Wirkung des Amantadins und seiner Salze überraschenderweise durch Kombination mit Selegilin oder Salzen des Selegilins synergistisch gesteigert wird.

Aufgabe der Erfindung ist die Bereitstellung eines verbesserten Arzneimittels zur Behandlung des Parkinsonismus.

Die Erfindung betrifft die Kombination der Wirkstoffe Amantadin und Selegilin, wobei diese Wirkstoffe auch in Form ihrer Salze mit physiologisch unbedenklichen Säuren vorliegen können. Die in der Beschreibung und in den Patentansprüchen angegebenen Gewichtsmengen beziehungsweise Gewichtsteile beziehen sich jeweils auf die reinen Wirkstoffe, das heißt nicht auf Salze dieser Wirkstoffe.

Amantadin und Selegilin werden vorzugsweise als Säureadditionssalze verwendet, wobei insbesondere die Salze mit Halogenwasserstoffsäuren (zum Beispiel das Hydrochlorid) oder organischen Säuren (zum Beispiel das Citrat) in Frage kommen.

Die erfindungsgemäße Kombination zeigt beispielsweise bei der Parkinsonschen Krankheit einen überraschenden Synergismus der Wirkung, die gegenüber der Wirkung des reinen Amantadins synergistisch gesteigert ist. Das Selegilin allein besitzt demgegenüber nahezu keine Antiparkinsonwirkung.

Als Versuchsmodell für eine Antiparkinson-Wirkung kann zum Beispiel folgendes in Frage kommen:

Tierversuche:

Die Substanzen, die als Antiparkinsonmittel in Frage kommen, werden anhand der Erhöhung der Dopamin-Konzentration beziehungsweise des Dopamin-Umsatzes (zum Beispiel Dopamin zu DOPAC = 3,4-Dihydroxyphenylessigsäure beziehungsweise Dopamin zu Homovanillinsäure = 3-Methoxy-4-hydroxyphenylessigsäure) im Striatum (Gehirnbereich im extrapyramidalen motorischen System; Bezeichnung für einen Teil der basalen Stammganglien) von mongolischen Springmäusen überprüft. Die präparierten Striatumproben werden auf Dopamin mittels Hochdruckflüssigkeitschromatographie (HPLC-ESA-ECD; siehe Felice et al, J., Neurochemistry 31 (1978) Seite 1461) und auf Homovanillinsäure und DOPAC mittels Hochdruckflüssigkeitschromatographie (HPLC-BAS-ECD; siehe Sperk, J. Neurochemistry 38 (1982) Seite 840) analysiert. Dabei werden Versuchstiere beiderlei Geschlechts ausgewählt mit einem Gewicht von 70 - 80 g.

Die Substanzen werden den Tieren intraperitoneal (in die Bauchhöhle) in Konzentrationen von 0,8 - 200 mg Selegininhydrochlorid pro ml beziehungsweise 3 - 40 mg Amantadinhydrochlorid pro ml injiziert. Gruppen von jeweils 5 Mäusen erhalten jeweils Amantadindosen von 0,5 - 400 mg/kg, beispielsweise 1 - 100 mg/kg und Selegilindosen von 0,04 - 200 mg/kg, beispielsweise 0,1 - 50 mg/kg zur Ermittlung der ED 50-Werte.

Für die Ermittlung des synergistischen Effektes werden jeweils verschiedene Bruchteile beziehungsweise Vielfache (zum Beispiel vom 0,01-fachen bis zum 2-fachen) der Selegilin-ED 50-Dosis kombiniert. Zu verschiedenen Zeiten (beispielsweise nach 0,5 - 6 Stunden) nach Applikation der Einzelsubstanzen beziehungsweise der Kombination der Einzelsubstanzen werden die Tiere getötet und die Striatum-Proben entnommen.

Mittels HPLC wird der Gehalt der homogenisierten Proben an Dopamin, DOPAC und Homovanillinsäure bestimmt.

Ein synergistischer Effekt kann sich dadurch zeigen, daß die Werte für Dopamin, DOPAC und Homovanillinsäure gegenüber den Werten bei Gabe der Einzelsubstanzen erhöht sind.

Untersuchungsmethodik am Menschen:

Der Effekt der Antiparkinson-Wirkung wird beispielsweise nach Webster, D.D., Med. Treat. (N.Y.) 5, 257 - 282 (1968) festgestellt. Hierbei werden nach Applikation der Monosubstanz Amantadin beziehungsweise der Kombination die verschiedenen Symptome zugeordnet und gewichtet:

1. verlangsamte Motorik der Hände
2. Rigor (Erhöhung des Muskeltonus)
3. vornübergebeugte Haltung
4. Mitschwingen der oberen Extremitäten
5. Gang
6. Tremor (Zittern)
7. Gesichtsausdruck
8. Seborrhoe
9. Sprache
10. Selbständigkeit.

Eine Antiparkinson-Wirkung ist dann vorhanden, wenn die Summe der sich aus der Webster-Skala ergebenen Werte (der sogenannte disability-score) gegenüber dem Ausgangswert ohne Medikation erniedrigt ist (< 30). Der synergistische Effekt der Kombination ergibt sich dadurch, daß bei zusätzlicher Verwendung von Selegilin die Summe des disability-scores gegenüber derjenigen, die bei alleiniger Anwendung von Amantadin erreicht würde, niedriger ist und/oder die Dauer der Wirksamkeit des Amantadins verlängert wird.

Die synergistische Wirkungssteigerung am Menschen ist besonders ausgeprägt, wenn in der Kombination mindestens 20 mg Amantadin und mindestens 1 mg Selegilin vorliegen.

Synergistischer Effekt bei der Anwendung der erfindungsgemäßen Kombination in den Frühstadien des Morbus Parkinsons:

Eine möglichst frühzeitige Verabreichung des Monoaminoxidase-B-Hemmers Selegilin ist aus Gründen der Progressionshemmung der Erkrankung sinnvoll. Da Selegilin allein keine ausreichende Wirkung in Bezug auf Besserung der Parkinson-Symptomatik aufweist, zeigt sich überraschend, daß nach gleichzeitiger Gabe von Amantadin dessen Wirksamkeit synergistisch gesteigert wird (Erniedrigung des Webster-Summen-course). Damit wird dem Patienten erstmals eine Therapiemöglichkeit in der Frühphase seiner Erkrankung eröffnet, die das Fortschreiten des Morbus Parkinsons verlangsamt, die Gesamtsymptomatik zufriedenstellend bessert und gleichzeitig die Nebenwirkungsrate erstaunlich gering hält.

Synergistischer Effekt der erfindungsgemäßen Kombination bei krisenhaften Verschlechterungen des Morbus Parkinsons:

Die relativ langsam einsetzende Besserung der akinetischen Krise nach intravenöser Gabe von Amantadin konnte überraschend durch Zugabe von Selegilin beschleunigt werden. Die danach beobachtete Wirkung ist ausgeprägter und länger anhaltend als nach alleiniger Gabe der Einzelkomponenten.

Für die erfindungsgemäßen Kombinationen kommen zum Beispiel folgende Indikationen in Betracht: Morbus Parkinson in allen Krankheitsstadien, Depressionen, Narkolepsie, Hirnorganisches Psychosyndrom.

Die Tagesdosen der erfindungsgemäßen Kombination bestehen zum Beispiel aus 20 bis 4000 mg, vorzugsweise 50 bis 3000 mg und insbesondere 100 bis 2000 mg Amantadin und 1 bis 250 mg, vorzugsweise 3 bis 200 mg, insbesondere 4 bis 150 mg Selegilin.

Die Tagesdosen können in Form einer einmaligen Verabreichung der gesamten Menge oder in Form von 1 bis 5, insbesondere 1 bis 4 Teildosen pro Tag eingesetzt werden. Im allgemeinen ist eine Verabreichung 1 bis 3 mal, insbesondere 1 bis 2 mal täglich bevorzugt. Beispielsweise beträgt die bevorzugte Dosis für die Kombination von Amantadin und Selegilin vorzugsweise 100 bis 400 mg Amantadin und 4 bis 30 mg Selegilin 1 bis 3 mal täglich. Insbesondere beträgt diese Dosis etwa 250 mg Amantadin und etwa 10 mg Selegilin 1 bis 3 mal täglich.

Amantadin und Selegilin liegen in einer Dosierungseinheit beispielsweise in folgendem Gewichtsverhältnis vor:
1 Gewichtsteil Selegilin wird zum Beispiel mit 0,4 - 800 Gewichtsteilen Amantadin, vorzugsweise 1 Gewichtsteil Selegilin mit 1,2 - 200 Gewichtsteilen Amantadin, insbesondere 1 Gewichtsteil Selegilin mit 3,3 - 100 Gewichtsteilen Amantadin kombiniert.

Beispielsweise lassen sich für die Kombination 20 - 800 mg Amantadin und 1 - 50 mg Selegilin, vorzugsweise 50 - 600 mg Amantadin und 3 - 40 mg Selegilin, insbesondere 100 - 400 mg Amantadin und 4 - 30 mg Selegilin, speziell 200 - 300 mg Amantadin und 5 - 15 mg Selegilin leicht zum Arzneimittel formulieren.

Diese zuvor angegebenen Gewichtsmengen gelten nur für homogene Mischungen von Amantadin und Selegilin (zum Beispiel Suppositorien oder Einschichttablette). Bei anderen Formulierungen, zum Beispiel Kapseln und Zweischichttabletten, können die Komponenten natürlich auch in anderen Gewichtsmengen zusammen kombiniert werden.

Die Dosierungseinheit der erfindungsgemäßen Kombination kann beispielsweise enthalten:

a) Bei peroralen Arzneiformen:
20 - 800 mg Amantadin, vorzugsweise 50 - 600 mg, insbesondere 100 - 400 mg Amantadin und 1 - 50 mg, vorzugsweise 3 - 40 mg, insbesondere 4 - 30 mg Selegilin.
Diese Dosen können beispielsweise 1 bis 5, vorzugsweise 1 bis 4, insbesondere 1 bis 3 mal täglich verabreicht werden.

b) Bei parenteralen Arzneiformen (zum Beispiel intravenös, intramuskulär):
20 - 800 mg Amantadin, vorzugsweise 50 - 600 mg, insbesondere 100 - 400 mg Amantadin und 1 - 50 mg, vorzugsweise 3 - 40 mg, insbesondere 4 - 30 mg Selegilin.
Diese Dosen können beispielsweise 1 bis 5, vorzugsweise 1 bis 4, insbesondere 1 bis 3 mal täglich verabreicht werden.

c) Bei Arzneiformen zur rektalen oder vaginalen Applikation:
20 - 800 mg Amantadin, vorzugsweise 50 - 600 mg, insbesondere 100 - 400 mg Amantadin und 1 - 50 mg, vorzugsweise 3 - 40 mg, insbesondere 4 - 30 mg Selegilin.
Diese Dosen können beispielsweise 1 bis 5, vorzugsweise 1 bis 4, insbesondere 1 bis 3 mal täglich verabreicht werden.

d) Bei Arzneiformen zur Applikation auf die Haut und Schleimhäute (zum Beispiel als Lösungen, Lotionen, Emulsionen, Salben, Pflaster und so weiter):
20 - 800 mg Amantadin, vorzugsweise 50 - 600 mg, insbesondere 100 - 400 mg Amantadin und 1 - 50 mg, vorzugsweise 3 - 40 mg, insbesondere 4 - 30 mg Selegilin.
Diese Dosen können beispielsweise 1 bis 5, vorzugsweise 1 bis 4, insbesondere 1 bis 3 mal täglich verabreicht werden.

Selbstverständlich können auch galenische Zubereitungen hergestellt werden, welche die oben angegebenen Dosierungseinheiten 2 bis beispielsweise 6 mal enthalten.

Die oben angegebenen Dosen und Gewichtsteile, die sich auf die Anwendung am Menschen beziehen, sind jeweils bezogen auf die freien Basen.

Die akute Toxizität der erfindungsgemäßen Kombination an der Maus (ausgedrückt durch die LD 50 mg/kg; Methode: Litchfield und Wilcoxon, J. Pharmacol. Exper. Ther. 95 : 99, 1959) liegt beispielsweise für die Kombination Amantadin (HCl-Salz) und Selegilin (HCl-Salz) (Gewichtsverhältnis 10 : 1) bei oraler Applikation zwischen 600 - 700 mg/kg.

Die erfindungsgemäße Kombination ist zur Herstellung pharmazeutischer Zusammensetzungen und Zubereitungen geeignet. Die pharmazeutischen Zusammensetzungen beziehungsweise Arzneimittel enthalten als Wirkstoff die erfindungsgemäße Kombination in einer Formulierung. Die Einzelwirkstoffe der Kombination können aber auch in jeweils getrennten Formulierungen vorliegen, wobei die bereits angegebenen Wirkstoffmengen jeweils für die betreffende Dosierungseinheit verwendet werden. Die Wirkstoffe beziehungsweise Wirkstoffkombination liegen gegebenenfalls in Mischung mit anderen pharmakologisch beziehungsweise pharmazeutisch wirksamen Stoffen vor.

Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor KG, Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumen samenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit oder Mannit, die gegebenenfalls auch verethert sein können, Ester der Citronensäure mit primären Alkoholen und Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat und Magnesiumcarbonat.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel Polyacrylsäureester oder Celluloseether.

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxyd, Fettalkohole, Triglyceride, Partialester des Glycerins und Paraffine. Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierte Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnussöl, Rhizinusöl, Sesamöl, Baumwollsaatöl, Maisöl.

Siehe auch Dr. H.P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der Wirkstoffe erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, peroral, enteral, pulmonal, rectal, nasal, vaginal, lingual, intravenös, intraarteriell, intrakardial, intramuskulär, intraperitoneal, intracutan, subcutan. Bei den parenteralen Zubereitungsformen handelt es sich insbesondere um sterile beziehungsweise sterilisierte Erzeugnisse.

Die erfindungsgemäße Kombination kann auch als ein Erzeugnis vorliegen, bei dem jeweils die beiden Einzelwirkstoffe in getrennten Formulierungen vorliegen, so daß auch eine getrennte oder auch zeitlich abgestufte Verabreichung möglich ist.

Falls solche getrennten Formulierungen vorliegen, sind diese aufeinander abgestimmt und enthalten die jeweiligen Wirkstoffe in der Dosierungseinheit in denselben Mengen und entsprechenden Gewichtsverhältnissen, in denen sie in der kombinierten Mischung vorliegen können.

Bei getrennter Anwendung ist es auch möglich, daß beide Kombinationspartner nicht gleichzeitig verabreicht werden. In solchen Fällen kann zum Beispiel das Selegilin 1 mal pro Tag gegeben werden (Dosis zum Beispiel 5 - 30 mg) und das Amantadin gleichzeitig (Dosis zum Beispiel 50 - 200 mg) und dann 3 bis 4 weitere Dosen (zum Beispiel zwischen 50 - 200 mg) im Abstand von jeweils 4 Stunden.

In den flüssigen Zubereitungen liegt das Amantadin beispielsweise in Konzentrationen von 2 - 10, vorzugsweise 4 - 6, insbesondere 5 Gewichtsprozent und das Selegilin in Konzentrationen von 0,1 - 1, vorzugsweise 0,3 - 0,5, insbesondere 0,375 Gewichtsprozent vor.

Für Zubereitungen, bei denen die erfindungsgemäße Kombination im geschmolzenem Hartfett suspendiert und homogenisiert wird, werden beispielsweise pro 1 Gewichtteil Amantadin (in der Kombination) oder pro 1 Gewichtsteil Selegilin (falls das Selegilin als getrennte Formulierung vorliegt) 10 - 50 Gewichtsteile Hartfett verwendet, wobei gegebenenfalls auch 0,01 - 1 Gewichtsteile (bezogen auf 1 Gewichtsteil Amantadin) Sojalecithin zusätzlich beigemischt werden können.

Für die Herstellung von Zubereitungen in Form von Tabletten oder Kapseln werden zum Beispiel pro 1 Gewichtsteil Amantadin (in der Kombination) oder pro 1 Gewichtsteil Selegilin (falls das Selegilin als getrennte Formulierung vorliegt) 0,01 - 0,5 Gewichtsteile Stärke oder Gelatine oder 0,005 - 0,3 Gewichtsteile Vinylpyrrolidon-Vinylacetat-Copolymerisat zur Granulierung verwendet. Gegebenenfalls können vorher pro 1 Gewichtsteil Amantadin (in der Kombination) oder pro 1 Gewichtsteil Selegilin (falls das Selegilin als getrennte Formulierung vorliegt) 0,01 - 10 vorzugsweise 0,05 - 1 Gewichtsteile Calciumhydrogenphosphat zugemischt werden. Das erhaltene Granulat wird beispielsweise mit Luft, deren Temperatur zwischen 50 - 70°C liegt, getrocknet.

Das trockene Granulat kann beispielsweise noch mit 0,005 bis 0,1 Gewichtsteilen Magnesiumstearat, 0,001 - 0,1 Gewichtsteilen Siliziumdioxid und/oder 0,05 - 2 Gewichtsteilen Stärke homogen vermischt werden (die zuvor angegebenen Gewichtsteile beziehen sich jeweils pro 1 Gewichtsteil Amantadin (in der Kombination) oder pro 1 Gewichtsteil Selegilin (falls das Selegilin als getrennte Formulierung vorliegt).

Beispiele

Beispiel 1

Injektionslösung mit 100 mg Amantadinhydrochlorid und 7,5 mg Selegilinhydrochlorid

25 g Amantadinhydrochlorid und 1,875 g Selegilinhydrochlorid werden nacheinander in 450 ml mit Stickstoff begastem Wasser für Injektionszwecke gelöst. Die Lösung wird mit Stickstoff begastem Wasser für Injektionszwecke auf 500 ml aufgefüllt und nach sorgfältigem Mischen und unter weiterer Stickstoffbegasung durch ein Membranfilter der Porenweite 0,2 um mit Glasfaservorfilter steril filtriert. Das Filtrat wird unter aseptischen Bedingungen sowie unter Stickstoffbegasung in farblose Ampullen, Inhalt 2 ml, abgefüllt. 2 ml der Lösung enthalten 100 mg Amantadinhydrochlorid und 7,5 mg Selegilinhydrochlorid.

Beispiel 2

Kapseln mit 100 mg Amantadinhydrochlorid und 7,5 mg Selegilinhydrochlorid

10 kg Amantadinhydrochlorid werden unter Verwendung eines geeigneten Mischers mit 0,75 kg Selegilinhydrochlorid intensiv gemischt. Danach wird die Mischung in einer Wirbelschicht-Sprühgranulationsapparatur mit einer Lösung aus 0,25 kg Gelatine in 2,25 kg Wasser in bekannter Weise granuliert. Nach Zumischen von 0,85 kg Maisstärke, 0,1 kg Magnesiumstearat und 0,05 kg hochdispersem Siliziumdioxid wird die Mischung in einer Füllmenge von jeweils 120 mg in Hartgelatinekapseln der Größe 2 gefüllt. Eine Kapsel enthält 100 mg Amantadinhydrochlorid und 7,5 mg Selegilinhydrochlorid.

**Patentansprüche für die Vertragsstaaten: AT, BE, CH, DE, FR, GB, IT, LI, LU, NL, SE**

1. Erzeugnis zur Bekämpfung der Parkinsonschen Krankheit, von Depressionen, der Narkolepsie und dem hirnorganischen Psychotydiom, dadurch gekennzeichnet, daß es neben den üblichen pharmazeutischen Träger- und/oder Verdünnungsbeziehungsweise Hilfsstoffen Amantadin und Selegilin oder jeweils ein physiologisch unbedenkliches Salz beider Wirkstoffe in einer eine synergistische Wirkung erzeugenden Menge enthält und in einer Form vorliegt, die sowohl eine gemeinsame als auch eine getrennte therapeutische Anwendung beider Wirkstoffe gestattet.

2. Erzeugnis nach Anspruch 1, dadurch gekennzeichnet, daß in der Kombination auf ein Gewichtsteil Selegilin jeweils 0,4 bis 800 Gewichtsteile Amantadin kommen.

3. Erzeugnis nach einem oder mehreren der vorangegangenen Ansprüche, dadurch gekennzeichnet, daß in der Dosierungseinheit die Kombination 20 bis 800 mg, vorzugsweise 50 bis 600 mg, Amantadin und 1 bis 50 mg, vorzugsweise 3 bis 40 mg, Selegilin enthält.

4. Verfahren zur Herstellung eines Erzeugnisses nach einem oder mehreren der vorangegangenen Ansprüche, dadurch gekennzeichnet, daß man 1 Gewichtsteil Selegilin und 0,4 bis 800 Gewichtsteile Amantadin, wobei die Wirkstoffe auch in Form ihrer physiologisch unbedenklichen Salze vorliegen können, zusammen mit üblichen Träger- und/oder Verdünnungs- beziehungsweise Hilfsstoffen zu einem Erzeugniss verarbeitet, welches der Dosierungseinheit 20 bis 800 mg Amantadin und 1 bis 50 mg Selegilin enthält.

5. Verfahren zur Herstellung eines Erzeugnisses nach einem oder mehreren der vorangegangenen Ansprüche, dadurch gekennzeichnet, daß man 1 Gewichtsteil Selegilin und 0,4–800 Gewichtsteile Amantadin oder deren Salze mit physiologisch unbedenklichen Säuren zusammen mit üblichen Träger- und/oder Verdünnungs- beziehungsweise Hilfsstoffen bei Temperaturen zwischen 20 und 80°C vermischt beziehungsweise homogenisiert, die so erhaltene Mischung zur Herstellung von Zubereitungen, die in der Dosierungseinheit 20–800 mg Amantadin und 1–50 mg Selegilin enthalten, in Hohlzellen entsprechender Größe ausgießt oder in Kapseln entsprechender Größe abfüllt oder zu Tabletten verpreßt oder granuliert und dann gegebenenfalls unter Zusatz von weiteren üblichen Hilfsstoffen in Kapseln füllt oder zu Tabletten verpreßt oder unter Verwendung physiologisch unbedenklicher Lösungsmittel Lösungen beziehungsweise Dispersionen herstellt, wobei in solchen flüssigen Zubereitungen die Konzentration an Amantadin 2–10 Gewichtsprozent und an Selegilin 0, 1–1 Gewichtsprozent ist.

6. Verfahren zur Herstellung eines Erzeugnisses nach einem oder mehreren der vorangegangenen Ansprüche, dadurch gekennzeichnet, daß man 1 Gewichtsteil Selegilin und 0,4–800 Gewichtsteile Amantadin oder deren Salze mit physiologisch unbedenklichen Säuren, gegebenenfalls mit mindestens einem der Hilfsstoffe Stärke, Cellulose, Calciumhydrogenphosphat und modifizierte Stärke vermischt, mit einer wässrigen Gelatinelösung oder Stärkelösung oder einem wässrigen Vinylpyrrolidon-Vinylacetat Copolymerisat granuliert und das so erhaltene Granulat mit Magnesiumstearat und hochdispersem Siliziumdioxid sowie gegebenenfalls auch Stärke und/oder Cellulose vermischt und zu Tabletten verpreßt oder in Kapseln abfüllt.

7. Verfahren zur Herstellung eines Erzeugnisses nach einem oder mehreren der vorangegangenen Ansprüche, dadurch gekennzeichnet, daß man 1 Gewichtsteil Selegilin und 0,4–800 Gewichtsteile Amantadin oder deren Salze mit physiologisch unbedenklichen Säuren, gegebenenfalls nach Zusatz von Sojalecithin, bei Temperaturen zwischen 33–37°C in geschmolzenem Hartfett suspendiert und homogenisiert und anschließend die Mischung in Hohlzellen ausgießt.

8. Gemeinsame Verwendung von Amantadin und Selegilin zur Herstellung von Erzeugnissen nach einem oder mehreren der vorangegangenen Ansprüche.

**Patentansprüche für die Vertragsstaaten : ES, GR**

1. Verfahren zur Herstellung eines Erzeugnisses zur Bekämpfung der Parkinsonschen Krankheit, von Depressoinen, der Narkolepsie und dem hirnorganischen Psychotydtom, welches Amantadin und Selegilin oder jeweils ein physiologisch unbedenkliches Salz beider Wirkstoffe in einer eine synergistische Wirkung erzeugenden Menge enthält und in einer Form vorliegt, die sowohl eine gemeinsame als auch eine getrennte therapeutische Anwendung beider Wirkstoffe gestattet, dadurch gekennzeichnet, daß man 1 Gewichtsteil Selegilin und 0, 4 bis 800 Gewichtsteile Amantadin wobei die Wirkstoffe auch in Formihrer physiologisch unbedenklichen Salze vorliegen können, zusammen mit üblichen Träger- und/oder Verdünnungs- beziehungsweise Hilfsstoffen zu einem Erzeugniss verarbeitet, welches in der Dosierungseinheit 20 bis 800 mg Amantadin und 1 bis 50 mg Selegilin enthält.

2. Verfahren zur Herstellung eines Erzeugnisses nach Anspruch 1, dadurch gekennzeichnet, daß man 1 Gewichtsteil Selegilin und 0,4–800 Gewichtsteile Amantadin oder deren Salze mit physlologisch unbedenklichen Säuren zusammen mit üblichen Träger- und/oder Verdünnungs- beziehungsweise Hilfsstoffen bei Temperaturen zwischen 20 und 800°c vermischt beziehungsweise homogenisiert, die so erhaltene Mischung zur Herstellung von Zubereitungen, die in der Dosierungseinheit 20–800 mg Amantdin und 1–50 mg Selegilin enthalten, in Hohlzellen entsprechender Größe ausgießt oder in Kapseln entsprechender Größe abfüllt oder zu Tabletten verpreßt oder granuliert und dann gegebenenfalls unter Zusatz von weiteren üblichen Hilfsstoffen in Kapseln füllt oder zu Tabletten verpreßt oder unter Verwendung physiologisch unbedenklicher Lösungsmittel Lösungen beziehungsweise Dispersionen herstellt, uobei in soLchen flüssigen Zubereitungen die Konzentration an Amantadin 2 - 10 Gewichtsprozent und an SelegiLin 0, 1 - 1 Gewichtsprozent ist.

3. Verfahren zur Herstellung eines Erzeugnisses nach einem oder mehreren der vorangegangenen Ansprüche, dadurch gekennzeichnet, daß man 1 Gewichtsteil Selegilin und 0,4–800 Gewichtsteile Amantadin oder deren Salze mit physiologisch unbedenklichen Säuren gegebenenfalls mit mindestens einem der Hilfsstoffe Stärke, Cellulose, Calciumhydrogenphosphat und modifizierte Stärke vermischt, mit einer wässrigen Gelatinelösung oder Stärkelösung oder einem wässrigen Vinylpyrrolidon Vinylacetat Copolymerisat granuliert und das erhaltene Granulat mit Magnesiumstearat und so hochdispersem Siliziumdioxid sowie gegebenenfalls auch Stärke und/oder Cellulose vermischt und zu Tabletten verpreßt oder in Kapseln abfüllt.

4. Verfahren zur Herstellung eines Erzeugnisses nach einem der mehreren der vorangegangenen Ansprüche, dadurch gekennzeichnet, daß man 1 Gewichtsteil Selegilin und 0, 4 - 800 Gewichtsteile Amantadin oder deren Salze mit physiologisch unbedenklichen Säuren gegebenenfalls nach Zusatz von Sojalecithin, bei Temperaturen zwischen 33–37°C in geschmolzenem Hartfett suspendiert und homogenisiert und anschließend die Mischung in HohLzellen ausgießt.

**Claims for the contracting states: AT, BE, CH, DE, FR, GB, IT, LI, LU, NL, SE**

1. A product for the treatment of Parkinson's disease, depression, narcolepsy and cerebro-organic psychotydioma, characterized in that, in addition to the usual pharmaceutical excipients and/or diluents or auxiliaries, it contains amantadine and/or selegiline or a physiologically acceptable salt thereof in a quantity which produces a synergistic effect and is present in a form which enables the two active substances to be therapeutically applied both together and also separately.

2. A product as claimed in claim 1, characterized in that the combination contains 0.4 to 800 parts by weight amantadine to 1 part by weight selegiline.

3. A product as claimed in one or more of the preceding claims, characterized in that the combination contains 20 to 800 mg and preferably 50 to 600 mg amantadine and 1 to 50 mg and preferably 3 to 40 mg selegiline in the dosage unit.

4. A process for the preparation of the product claimed in one or more of the preceding claims, characterized in that 1 part by weight selegiline and 0.4 to 800 parts by weight amantadine, optionally in the form of their physiologically acceptable salts, are processed together with typical excipients and/or diluents or auxiliaries to form a product containing 20 to 800 mg amantadine and 1 to 50 mg selegiline in the dosage unit.

5. A process for the preparation of the product claimed in one or more of the preceding claims, characterized in that 1 part by weight selegiline and 0.4 to 800 parts by weight amantadine or salts thereof with physiologically acceptable acids are mixed or homogenized together with typical excipients and/or diluents or auxiliaries at temperatures in the range from 20 to 80°C, the resulting mixture – for the production of preparations containing 20 to 800 mg amantadine and 1 to 50 mg selegiline in the dosage unit – is poured out into hollow cells of corresponding size or packed into capsules of corresponding size or compressed to tablets or granulated and then packed into capsules or compressed to tablets, optionally with addition of other typical auxiliaries, or solutions or dispersions are prepared using physiologically acceptable solvents, the concentrations of amantadine and selegiline in such liquid preparations being 2–10% by weight and 0.1–1% by weight, respectively.

6. A process for the preparation of the product claimed in one or more of the preceding claims, characterized in that 1 part by weight selegiline and 0.4 to 800 parts by weight amantadine or salts thereof with physiologically acceptable acids, optionally mixed with at least one of the auxiliaries starch, cellulose, calcium hydrogen phosphate and modified starch, are granulated with an aqueous gelatine solution or starch solution or an aqueous vinyl pyrrolidone/vinyl acetate copolymer and the granulate thus obtained is mixed with magnesium stearate and highly disperse silicon dioxide and, optionally, with starch and/or cellulose and the resulting mixture is compressed to tablets or packed in capsules.

7. A process for the preparation of the product claimed in one or more the preceding claims, characterized in that 1 part by weight selegiline and 0.4 to 800 parts by weight amantadine or salts thereof with physiologically acceptable acids are suspended and homogenized in molten hard fat at temperatures of 33 to 37°C, optionally after the addition of soybean lecithin, and the mixture is subsequently poured out into hollow cells.

8. The use of amantadine and selegiline together for the preparaton of the products claimed in one or more of the preceding claims.

**Claims for the contracting states: ES, GR**

1. A process for the production of a product for the treatment of Parkinson's disease, depression, narcolepsy and cerebro-organic psychotydioma, which contains amantadine and selegiline or a physiologically acceptable salt thereof in a quantity which produces a synergistic effect and is present in a form which enables the two active substances to be therapeutically applied both together and also separately, characterized in that 1 part by weight selegiline and 0.4 to 800 parts by weight amantadine, optionally in the form of their physiologically acceptable salts, are processed together with typical excipients and/or diluents or auxiliaries to form a product containing 20 to 800 mg amantadine and 1 to 50 mg selegiline in the dosage unit.

2. A process for the preparation of a product as claimed in claim 1, characterized in that 1 part by weight selegiline and 0.4 to 800 parts by weight amantadine or salts thereof with physiologically acceptable acids are mixed or homogenized together with typical excipients and/or diluents or auxiliaries at temperatures in the range from 20 to 80°C, the resulting mixture – for the production of preparations containing 20 to 800 mg amantadine and 1 to 50 mg selegiline in the dosage unit – is poured out into hollow cells of corresponding size or packed into capsules of corresponding size or compressed to tablets or granulated and then packed into capsules or compressed to tablets, optionally with addition of other typical auxiliaries, or solutions or dispersions are prepared using physiologically acceptable solvents, the concentrations of amantadine and selegiline in such liquid preparations being 2–10% by weight and 0.1–1% by weight, respectively.

3. A process for the preparation of a product as claimed in one or more of the preceding claims, characterized in that 1 part by weight selegiline and 0.4 to 800 parts by weight amantadine or salts thereof with physiologically acceptable acids, optionally mixed with at least one of the auxiliaries starch, cellulose, calcium hydrogen phosphate and modified starch, are granulated with an aqueous gelatine solution or starch solution or an aqueous vinyl pyrrolidone/vinyl acetate copolymer and the granulate thus obtained is mixed with magnesium stearate and highly disperse silicon dioxide and, optionally, with starch and/or cellulose and the resulting mixture is compressed to tablets or packed in capsules.

4. A process for the preparation of a product as claimed in one or more of the preceding claims, characterized in that 1 part by weight selegiline and 0.4 to 800 parts by weight amantadine or salts thereof with physiologically acceptable acids are suspended and homogenized in molten hard fat at temperatures of 33 to 37°C, optionally after the addition of soybean lecithin, and the mixture is subsequently poured out into hollow cells.

**Revendications pour les Etats contractants: AT, BE, CH, DE, FR, GB, IT, LI, LU, NL, SE**

1. Produit utilisable dans la lutte contre la maladie de Parkinson, les dépressions, la narcolepsie et le psychosyndrome organocérébral, caractérisé en ce qu'il contient, outre les excipients et/ou diluants ou adjuvants pharmaceutiques usuels, de l'amantadine et de la sélégiline ou un sel physiologiquement acceptable de chacune de ces deux substances actives dans une quantité qui produit un effet synergique, et en ce qu'il se présente sous une forme qui permet aussi bien une application thérapeutique commune que séparée des deux substances actives.

2. Produit selon la revendication 1, caractérisé en ce qu'il entre chaque fois dans la combinaison, pour 1 partie en poids de sélégiline, de 0,4 à 800 parties en poids d'amantadine.

3. Produit selon la revendication 1 ou 2, caractérisé en ce que la combinaison contient, dans l'unité posologique, de 20 à 800 mg, de préférence de 50 à 600 mg d'amantadine et de 1 à 50 mg, de préférence de 3 à 40 mg de sélégiline.

4. Procédé de préparation d'un produit selon l'une quelconque des revendications 1 à 3, caractérisé en ce qu'on travaille 1 partie en poids de sélégiline et 0,4 à 800 parties en poids d'amantadine, ces substances actives pouvant aussi se présenter sous forme de leurs sels physiologiquement acceptables, avec des excipients et/ou diluants ou adjuvants usuels, pour former un produit qui contient, dans l'unité posologique, de 20 à 800 mg d'amantadine et de 1 à 50 mg de sélégiline.

5. Procédé de préparation d'un produit selon l'une quelconque des revendications 1 à 3, caractérisé en ce qu'on mélange ou homogénéise avec des excipients et/ou diluants ou adjuvants usuels, à des températures comprises entre 20 et 80°C, 1 partie en poids de sélégiline et 0,4 à 800 parties en poids d'amatadine ou de leurs sels avec des acides physiologiquement acceptables et, pour la confection de préparations qui contiennent de 20 à 800 mg d'amatadine et de 1 à 50 mg de sélégiline dans l'unité posologique, on verse le mélange ainsi obtenu dans des cellules creuses de dimensions appropriées ou on en remplit des capsules de dimensions appropriées ou on le presse en comprimés ou on le granule, puis, avec addition éventuelle d'autres adjuvants usuels, on en remplit des capsules ou on le presse en comprimés, ou on prépare des solutions ou des dispersions en utilisant des solvants physiologiquement acceptables, les concentrations d'amatadine et de sélégiline dans de telles préparations liquides étant respectivement de 2 à 10% en poids et de 0,1 à 1% en poids.

6. Procédé de préparation d'un produit selon l'une quelconque des revendications 1 à 3, caractérisé en ce qu'on mélange 1 partie en poids de sélégiline et 0,4 à 800 parties en poids d'amantadine ou de leurs sels avec des acides physiologiquement acceptables, éventuellement avec au moins l'un des adjuvants amidon, cellulose, hydrogénophosphate de calcium et amidon modifié, on le granule avec une solution aqueuse de gélatine, une solution d'amidon ou un copolymère vinylpyrrolidone/acétate de vinyle aqueux, on mélange le granulé ainsi obtenu avec du stéarate de magnésium, du dioxyde de silicium fortement dispersé et éventuellement de l'amidon et/ou de la cellulose et on le presse en comprimés ou on en remplit des capsules.

7. Procédé de préparation d'un produit selon l'une quelconque des revendications 1 à 3, caractérise en ce qu'on met en suspension et on homogénéise dans une graisse dure fondue, à une température comprise entre 33 et 37°C, 1 partie en poids de sélégiline et 0,4 à 800 parties en poids d'amatadine ou de leurs sels avec des acides physiologiquement acceptables, éventuellement après addition de lécithine de soja, puis on verse le mélange dans ces cellules creuses.

8. Utilisation simultanée d'amantadine et de sélégiline pour la préparation de produits selon l'une quelconque des revendications 1 à 3.

**Revendications pour les Etats contractants: ES, GR**

1. Procédé de préparation d'un produit utilisable dans la lutte contre la maladie de Parkinson, les dépression, la narcolepsie et le psychosyndrome organocérébral, produit qui contient de l'amantadine et de la sélégiline ou un sel physiologiquement acceptable de chacune de ces deux substances actives dans une quantité produisant un effet synergique, et qui se présente sous une forme qui permet aussi bien une application thérapeutique commune que séparée des deux substances actives, caractérise en ce qu'on travaille 1 partie en poids de sélégiline et 0,4 à 800 parties en poids d'amatadine, ces substances actives pouvant aussi se présenter sous forme de leurs sels physiologiquement acceptables, avec des excipients et/ou diluants ou adjuvants usuels, pour former un produit qui contient, dans l'unité posologique, de 20 à 800 mg d'amatadine et de 1 à 50 mg de sélégiline.

2. Procédé de préparation d'un produit selon la revendication 1, caractérise en ce qu'on mélange ou homogénéise avec des excipients et/ou diluants ou adjuvants usuels, a des températures comprises entre 20 et 80°, 1 partie en poids de sélégiline et 0,4 à 800 parties en poids d'amatadine ou de leurs sels avec des acides physiologiquement acceptables et, pour la confection de préparations qui contiennent de 20 à 800 mg d'amantadine et de 1 à 50 mg de sélégiline dans l'unité posologique, on verse le mélange ainsi obtenu dans des cellules creuses de dimensions appropriées ou on en remplit des capsules de dimensions appropriées ou on le presse en comprimés ou on le granule, puis, avec addition éventuelle d'autres adjuvants usuels, on en remplit des capsules ou on le presse en comprimés, ou on prépare des solutions ou des dispersions en utilisant des solvants physiologiquement acceptables, les concentrations d'amatadine et de sélégiline dans de telles préparations liquides étant respectivement de 2 à 10% en poids et de 0,1 à 1% en poids.

3. Procédé de préparation d'un produit selon la revendication 1 ou 2, caractérisé en ce qu'on mélange 1 partie en poids de sélégiline et 0,4 à 800 parties en poids d'amatadine ou de leurs sels avec des acides physiologiquement acceptables, éventuellement avec du moins l'un des adjuvants amidon, cellulose, hydrogénophosphate de calcium et amidon modifié, on le granule avec une solution aqueuse de gélatine, une solution d'amidon ou un copolymére vinylpyrrolidone/acétate de vinyle aqueux, on mélange le granulé ainsi obtenu avec du stéarate de magnésium, du dioxyde de silicium fortement dispersé et éventuellement de l'amidon et/ou de la cellulose et on le presse en comprimés ou on en remplit des capsules.

4. Procédé de préparation d'un produit selon l'une quelconque des revendications 1 à 3, caractérisé en ce qu'on met en suspension et on homogénéise dans une graisse dure fondue, à une température comprise entre 33 et 37°C, 1 partie en poids de sélégiline et 0,4 à 800 parties en poids d'amatadine ou de leurs sels avec des acides physiologiquement acceptables, éventuellement après addition de lécithine de soja, puis on verse le mélange dans des cellules creuses.